# EUROPEAN PATENT APPLICATION

(11) **EP 4 799 728 A1**
(43) Date of publication of application: **02.09.2026**
(21) Application number: 25161113.3
(22) Date of filing: 28.02.2025
(51) Int. Cl.: B01L 3/00, B04B 13/00, G01N 33/49, B04B 9/10, G01N 33/574

(54) **MATERIAL SEPARATION DISC AND MATERIAL SEPARATION DEVICE COMPRISING THE SAME**

(30) Priority: 26.02.2025 KR 20250024939
(71) Applicant: CTCells, Inc., Daegu 42988 (KR)
(72) Inventor: KIM, Woon-Hae, 14345 Gyeonggi-do (KR); SHIN, Hyun Young, 16205 Gyeonggi-do (KR); LEE, Jung Min, 06307 Seoul (KR)
(74) Representative: Bjerkén Hynell KB

(57) **Abstract**

A material separation device (10) includes: a first motor (500) configured to rotate a material separation disk (100) including a chamber that is a sample storage space, a channel providing a passage for flow of a sample, and a valve for selectively opening and closing the channel around a rotation center on a rotation axis; a valve actuator (400) configured to supply energy for operating the valve; a third motor (700) configured to rotate the valve actuator (400) around a rotation center that is coaxial with the rotation center of the material separation disk (100) on a common rotation axis; and a controller (800) configured to control the first motor (500), the third motor (700), and the valve actuator (400) to supply energy to a valve material included in the valve while the material separation disk (100) and the valve actuator (400) rotate at different rotational speeds.

## Description

### [TECHNICAL FIELD]

The present disclosure relates to a blood separation disk and a material separation device including the blood separation disk.

### [DESCRIPTION OF THE RELATED ART]

Most deaths related to a malignant tumor are due to metastasis to tissues and organs distant from a point where the tumor first occurred. Therefore, early detection of metastasis is an important determining factor for the survival probability of cancer patients.

Diagnosis of cancer generally uses a diagnostic technique based on histopathology. Histopathology diagnostic techniques are techniques for diagnosing tumors by using tissue samples obtained from biopsies. This histopathology approach allows direct observation of tumor cells.

Meanwhile, circulating tumor cells (CTCs) are known to be discovered in patients before the tumor is first detected. Therefore, circulating tumor cells may play an important role in the early diagnosis and prediction of cancer. Because cancer is generally spread through blood, circulating tumor cells may be a marker for diagnosing whether cancer is metastasized. By using this, a disk-type device that extracts circulating tumor cells from samples, such as blood, is being researched and developed.

Such a material separation disk device rotates a disk body to generate centrifugal force, and uses the centrifugal force to separate circulating tumor cells from blood.

In the present disclosure, when configuring multiple chambers included in a material separation disk, the volume of a main chamber is specifically specified such that a blood layer may be accurately separated.

In addition, the material separation disk includes a plurality of chambers, and a valve that is opened and closed by external energy is arranged in a channel connecting respective chambers. The valve operates by receiving energy from the outside, and to operate the valve during a sample analysis process, rotation of the material separation disk is stopped, energy is supplied to the valve to melt a valve material, and then the material separation disk rotates again, and in a process of operating the valve, samples centrifuged into multiple layers are mixed again, which may reduce the reliability of analysis. In order to solve this problem, a related art (No. 10-2176587, title of the invention: Sample analysis method, and dynamic valve operating method) proposes a configuration in which a valve actuator that supplies energy to a valve rotates while rotating a material separation disk. A normal operation of a valve through efficient melting of a valve material is important for smooth fluid movement within a material separation disk. However, because there is a limitation in that a valve material is not completely melted when a shape of the valve does not match an energy emission portion while a material separation disk and a valve actuator rotate at the same speed and energy is emitted to the valve, this needs to be improved.

### [PRIOR ART DOCUMENTS]

Related art documents include Korean Patent Publication No. 10-2022-0135995 (Title of the invention: SPLIT DISK APPARATUS) and Korean Patent No. 10-2176587 (Title of the invention: Sample analysis method, and dynamic valve operating method).

### [DISCLOSURE OF THE INVENTION]

### [PROBLEMS TO BE SOLVED BY THE INVENTION]

The present disclosure provides a material separation disk which improves material separation characteristics by improving shapes of a main chamber and a sample chamber of the material separation disk, and a material separation device including the material separation disk.

Also, the present disclosure provides a material separation device capable of efficiently emitting energy to respective valves by adjusting a rotation speed of a valve actuator.

Technical problems to be solved by the present embodiment are not limited to the technical problems described above, and there may be other technical problems.

### [MEANS FOR SOLVING THE PROBLEMS]

A material separation disk coupled to a material separation device according to an embodiment includes a disk body that is rotatable, and at least one blood separator arranged on the disk body, including a plasma separation chamber, a main chamber, and a sample chamber, and including a first channel connecting the plasma separation chamber to the main chamber and a second channel connecting the main chamber to the sample chamber, and the main chamber has a cross-section of a preset shape when being cut parallel to a horizontal plane and has one side surface connected to the second channel, and a lower volume of the main chamber, which is determined by a height from a lower end of the cross-section of the main chamber to a point connected to the second channel, is formed to be less than or equal to a sum of a volume of a red blood cell layer of corresponding blood and volume of a density gradient solution under a condition that a mixture of the density gradient solution and blood corresponding to an entire volume of the main chamber is injected.

Also, a material separation device according to another embodiment includes a first motor configured to rotate a material separation disk including a chamber that is a sample storage space, a channel providing a passage for flow of a sample, and a valve for selectively opening and closing the channel, a valve actuator configured to supply energy for operating the valve, a third motor configured to rotate the valve actuator based on a coaxial axis and the first motor, and a controller configured to control the first motor, the third motor, and the valve actuator to supply energy to a valve material included in the valve while the material separation disk and the valve actuator rotate at different rotational speeds.

### [EFFECTS OF THE INVENTION]

According to an embodiment of the present disclosure, material separation characteristics may be improved by modifying shapes of a main chamber and a sample chamber of a material separation disk.

Also, a material separation disk and a valve actuator included in a material separation device may rotate together, and rotation speeds of the material separation disk and the valve actuator are configured differently such that energy of the valve actuator is efficiently emitted to all valves included in the material separation device, and thus, melting of a valve material may be improved.

### [BRIEF DESCRIPTION OF THE DRAWINGS]

FIG. 1 illustrates a configuration of a material separation device according to an embodiment of the present disclosure;
FIG. 2 illustrates a detailed configuration for movement of a valve actuator in a material separation device according to an embodiment of the present disclosure;
FIGS. 3 and 4 illustrate a detailed configuration of a material separation disk according to an embodiment of the present disclosure;
FIG. 5 illustrates a detailed configuration of a blood separator according to an embodiment of the present disclosure;
FIG. 6 illustrates a configuration of a main chamber of a material separation disk according to an embodiment of the present disclosure;
FIG. 7 illustrates a configuration of a main chamber of a material separation disk according to another embodiment of the present disclosure;
FIG. 8 illustrates a configuration of a sample chamber of a material separation disk according to an embodiment of the present disclosure;
FIG. 9 illustrates a configuration of a sample chamber of a material separation disk according to another embodiment of the present disclosure; and
FIG. 10 illustrates a rotation speed of a material separation device according to an embodiment of the present disclosure.

### [DETAILED DESCRIPTION OF THE INVENTION]

Hereinafter, embodiments of the present disclosure will be described in detail with reference to the attached drawings such that those skilled in the art to which the present disclosure belongs may easily practice the present disclosure. However, the present disclosure may be implemented in various different forms and is not limited to the embodiments described herein. In addition, in order to clearly describe the present disclosure in the drawings, parts that are not related to the description are omitted, and similar components are given similar reference numerals throughout the specification.

In the entire specification of the present disclosure, when a component is described to be "connected" to another component, this includes not only a case where the component is "directly connected" to another component but also a case where the component is "electrically connected" to another component with another element therebetween. In addition, when it is described that a portion "includes" a certain component, this means that the portion may further include another component without excluding another component unless otherwise stated, and it should be understood that the possibility of presence or addition of one or more other features, numbers, steps, operations, components, portions, or combinations thereof is not excluded in advance.

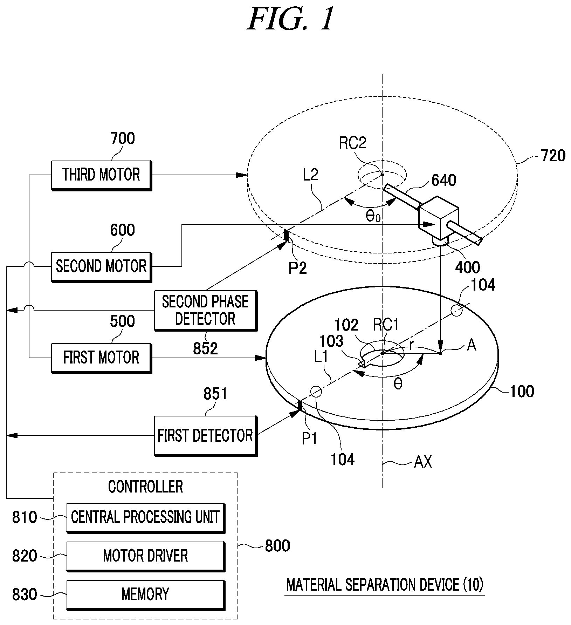

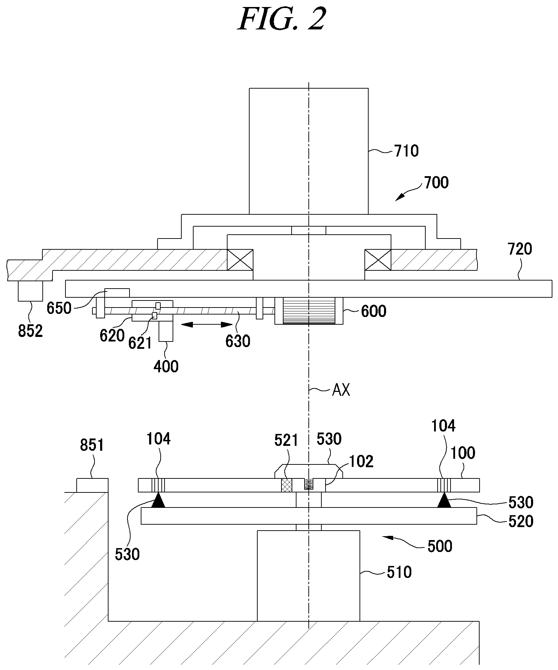

FIG. 1 illustrates a configuration of a material separation device according to an embodiment of the present disclosure, and FIG. 2 illustrates a detailed configuration for movement of a valve actuator in a material separation device according to an embodiment of the present disclosure.

Because a basic configuration corresponds to a configuration of patent No. 10-2176587 introduced above, descriptions are made below with reference to the patent.

A material separation device 10 includes a material separation disk 100, a valve actuator 400, a first motor 500 that rotates the material separation disk 100, a second motor 600 that adjusts a radial position of the valve actuator 400, and a third motor 700 that rotates the valve actuator 400.

The first motor 500 generates centrifugal force for centrifugal separation of a sample and movement of the sample by rotating the material separation disk 100. For example, a turntable 520 is provided on a rotation axis of the first motor 500 to which the material separation disk 100 is mounted. A mounting hole 102 corresponding to the turntable 520 is formed in the material separation disk 100. The mounting hole 102 has a form of a through-hole penetrating the center of the material separation disk 100 such that the turntable 520 may be inserted into the mounting hole 102. Also, a fixing pin 530 that is inserted into a fixing hole 104 formed in the material separation disk 100 may be formed to protrude on an outer periphery of the turntable 520. Each of the fixing hole 104 and the fixing pin 530 may be formed one or more, and as the fixing pin 530 is inserted into the fixing hole 104, the material separation disk 100 may be more stably fixed to the turntable 520. Although FIG. 2 illustrates that the fixing hole 104 and the fixing pin 530 are arranged along a first reference line L1 for the sake of convenience of description, positions of the fixing hole 104 and the fixing pin 530 may be arranged differently. Also, each of the number of fixing holes 104 and fixing pins 530 does not necessarily have to be two and may be one or three or more. In addition, unlike FIG. 2, the fixing hole 104 may be modified to be formed in the turntable 520, and the fixing pin 530 may be modified to correspond to the fixing hole 104 at a lower portion of the material separation disk 100.

In addition, the first motor 500 may also be directly connected to the turntable 520, and the first motor 500 may also be connected to the turntable by a power connection element such as a belt, chain, or gear.

The valve actuator 400 is an example of a valve actuator that supplies energy to a plurality of valves A of the material separation disk 100 to operate the plurality of valves A. The valve actuator 400 may emit electromagnetic waves, for example, laser light, to the plurality of valves A. The valve actuator 400 may be, for example, a laser diode.

The second motor 600 aligns the valve actuator 400 with the valve A. That is, the second motor 600 moves the valve actuator 400 in a radial direction of the material separation disk 100 to be located above the valve A. FIG. 2 illustrates an embodiment of the second motor 600. The second motor 600 is connected to a lead screw 630 that is rotated by the second motor 600, and a movement member 620 on which the valve actuator 400 is mounted may move along a radial direction of the rotation member 720 by the lead screw 630. The movement member 620 may be supported by, for example, a guide rail 640 extending in the radial direction of the material separation disk 100. The movement member 620 is provided with an engaging portion 621 that engages with a spiral groove of the lead screw 630. With this configuration, the movement member 620 moves in a radial direction along the guide rail 640 when the lead screw 630 rotates. As an example of a method of obtaining a radial reference position of the valve actuator 400, a method of utilizing a change in drive current of the second motor 600 may be used. For example, the movement member 620 is moved in a radial direction toward the inside or outside by using the second motor 600. When the movement member 620 moves to an end of the lead screw 630, the movement member 620 no longer moves, and in this case, a current that drives the second motor 600 changes rapidly. When a rapid change of the current is detected, it may be recognized that the valve actuator 400 is located at an inner end portion or outer end portion in the radial direction, and a controller 800 described below may recognize a position of the valve actuator 400 as a reference position in the radial direction of the valve actuator 400. As another method, a position detector 650 for detecting the reference position in the radial direction of the valve actuator 400 may be further provided. For example, the position detector 650 may detect the movement member 620 at any position in a radial direction. The position detector 650 may be, for example, a non-contact sensor, such as an optical sensor, or a contact sensor, such as a micro switch.

The material separation disk 100 may include a plurality of chambers for storing samples, channels for providing passages for flow of the samples, and the plurality of valves A for selectively opening and closing the channels. The material separation disk 100 may have a rotatable disk shape. The material separation disk 100 may include a lower structure including chambers each forming a fluid storage space and an engraved microfluidic structures such as channels providing passages for fluid between the chambers, and an upper structure (an upper plate) that is coupled to the lower structure and forms an upper wall of the microfluidic structure. The material separation disk 100 may have a two-plate structure in which the upper plate is coupled to a lower plate on which the microfluidic structure is formed. The upper plate may be bonded to the lower plate in various ways, such as bonding using an adhesive or double-sided adhesive tape, ultrasonic fusing, or laser fusing. The material separation disk 100 may be made of a plastic material, such as acrylic or polydimethylsiloxane (PDMS) that is easily molded and has a biologically inactive surface. However, the present disclosure is not limited thereto, and a material with chemical and biological stability, optical transparency, and mechanical processability may be used for the material separation disk 100.

Various types of the valve A may be provided in the material separation disk 100. For example, the valve A may be a closed valve that closes a channel in a normal state and opens the channel when receiving energy from the outside, an open valve that opens the channel in the normal state and closes the channel when receiving energy from the outside, and an on-off valve that may be switched between an open channel state, a closed channel state, and a reopen channel state.

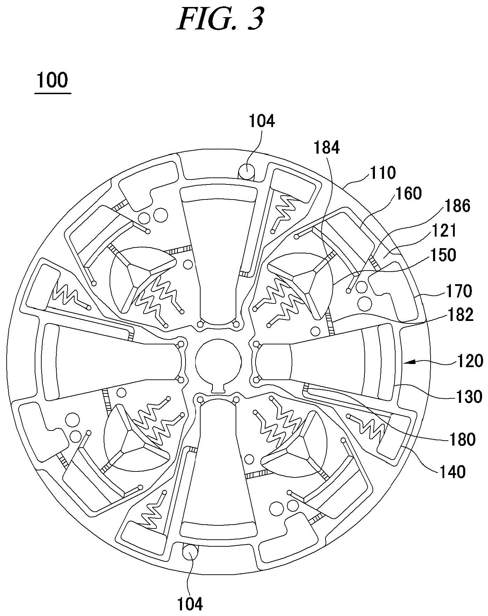

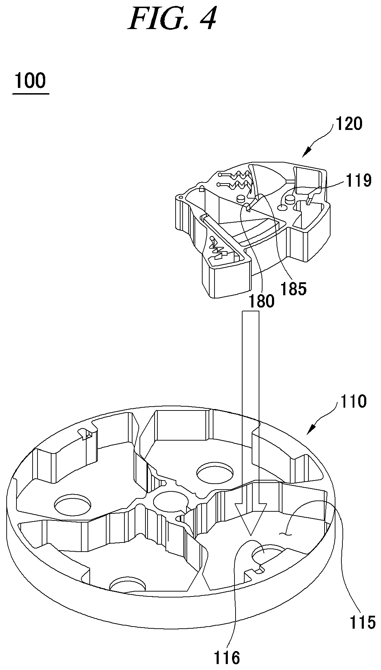

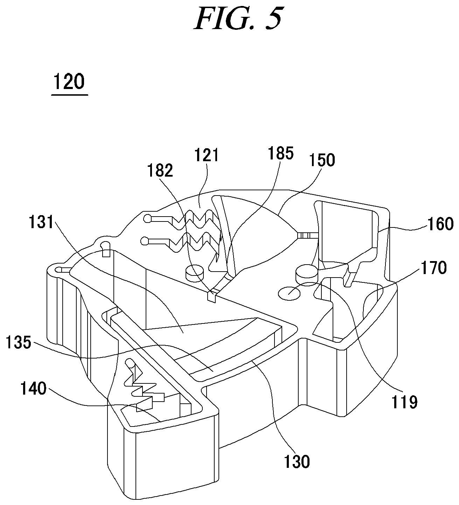

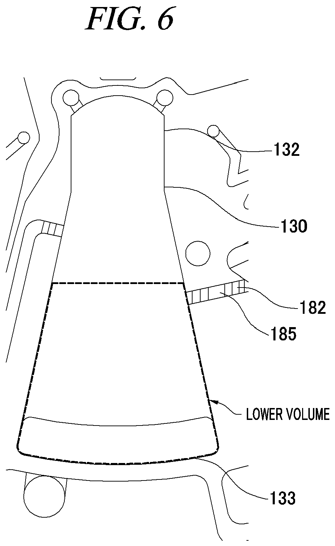

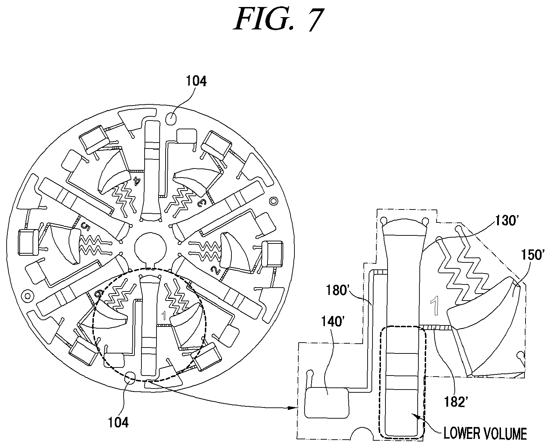

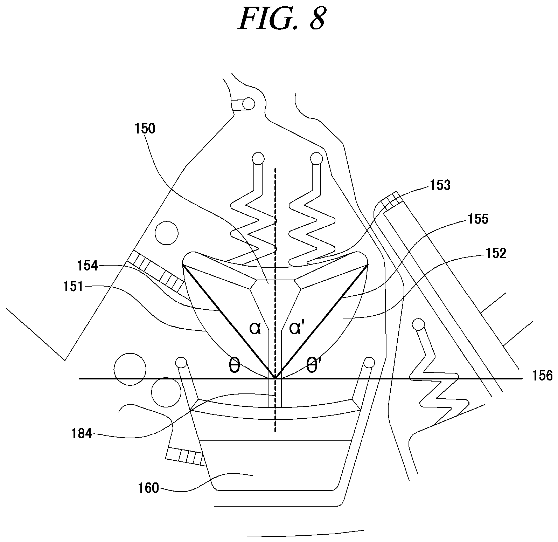

FIGS. 3 and 4 illustrate a detailed configuration of the material separation disk 100 according to an embodiment of the present disclosure, FIG. 5 illustrates a detailed configuration of a blood separator according to an embodiment of the present disclosure, FIG. 6 illustrates a configuration of a main chamber of a material separation disk 100 according to an embodiment of the present disclosure, FIG. 7 illustrates a configuration of a main chamber of a material separation disk according to another embodiment of the present disclosure, and FIG. 8 illustrates a configuration of a sample chamber of the material separation disk according to an embodiment of the present disclosure

The material separation disk 100 may include a disk body 110 that is rotatable and a plurality of blood separators (or at least one blood separator) 120 arranged in the disk body 110. The disk body 110 and the plurality of blood separators 120 may be integrally molded to form the material separation disk 100.

In another embodiment, the disk body 110 and the plurality of blood separators 120 may be formed separately from each other, and then the plurality of blood separators 120 may be respectively coupled to coupling grooves 115 formed in the disk body 110 to form the material separation disk 100. In this way, the plurality of blood separators 120 may each be implemented in the form of a modular split disk.

The material separation disk 100 may include a plurality of blood separators (or at least one blood separator) 120 that separates a target material, for example, a target cell, from a sample, for example, whole blood, by using centrifugal force generated when the disk body 110 rotates. Also, fixing holes (or at least one fixing hole) 104 may be formed in the disk body 110.

According to an embodiment, because the material separation disk 100 includes the disk body 110 and the blood separator 120 and has a separation structure in which the plurality of blood separators 120 are respectively coupled to the coupling grooves 115 of the disk body 110, over-fusion or partial fusion may be prevented from occurring depending on portions as in the related art, and thus, mass product deviation may be prevented and reproducibility may be increased.

The disk body 110 of the present embodiment has a circular disk shape, and a total of four coupling grooves 115 may be regularly provided in a radial direction. That is, the coupling grooves 115 of the same shape are formed at 90-degree intervals on the disk body 110, and a structure is provided in which the blood separator 120 having a modular separation disk shape corresponding to a shape of the coupling groove 115 is coupled to the coupling groove 115. The disk body 110 and the blood separator 120 may each be manufactured by injection molding, and accordingly, the disk body 110 and the blood separator 120 corresponding to each other in shape may be manufactured accurately and efficiently.

However, the number and shape of the coupling grooves 115 are not limited to the above-described example, and a method of manufacturing the disk body 110 and the blood separator 120 is not limited to the method described above. For example, the number of coupling grooves 115 may be 2 or 6, and it is obvious that the number of blood separators 120 corresponding to the number may be provided.

Referring to FIG. 4, by inserting the blood separator 120 into the coupling groove 115 of the disk body 110, the blood separator 120 may be easily coupled to the disk body 110, and then contact portions of the coupling groove 115 and the blood separator 120 are fused together by an ultrasonic fusion method, and thus, the coupling groove 115 may be more firmly coupled to the blood separator 120. However, in addition to the ultrasonic fusion method, a laser fusion method, a thermal fusion method, a hot air fusion method, and so on may be applied to fuse the coupling groove 115 to the blood separator 120.

In addition, a structure is used in which the blood separator 120 of the present embodiment is separated from the coupling groove 115, and for this purpose, a separation hole 116 may be formed in the bottom of the disk body 110 where the coupling groove 115 is formed, as illustrated in FIG. 4. In other words, when the blood separator 120 needs to be separated from the coupling groove 115, the blood separator 120 may be pressed in a direction of removal through the separation hole 116, and accordingly, the blood separator 120 may be easily separated from the coupling groove 115. Also, although not illustrated, an inner wall of the disk body 110 forming the coupling groove 115 and an outer wall of the blood separator 120 coupled to the inner wall may have tapered shapes corresponding to each other in shape. In other words, the inner wall of the disk body 110 forming the coupling groove 115 may have a tapered shape having a width decreasing toward a lower portion, and the outer wall of the blood separator 120 coupled to the coupling groove 115 may have a tapered shape having a width increasing toward a lower portion, and accordingly, the inner wall of the disk body 110 forming the coupling groove 115 may constantly correspond to the outer wall of the blood separator 120. Due to the tapered shape, the blood separator 120 may be prevented from being separated from the coupling groove 115 when the disk body 110 rotates.

Regarding a configuration of each blood separator 120, the blood separator 120 of the present embodiment includes a plurality of chambers, thereby being able to separate only target cells, that is, circulating tumor cells (CTCs) of the present embodiment, from whole blood.

The blood separator 120 of the present embodiment may include a separation disk body 121 and a plurality of chambers 130, 140, 150, 160, and 170 provided in the separation disk body 121 to separate target cells from whole blood by centrifugal force generated when the disk body 110 rotates.

The separation disk body 121 provides a basic frame and is provided in a shape corresponding to a shape of the coupling groove 115 described above.

The plurality of chambers 130, 140, 150, 160, and 170 may include a main chamber 130, a plasma separation chamber 140, a sample chamber 150, a separation chamber 160, and a storage chamber 170. Also, the blood separator 120 includes a first channel 180 connecting the plasma separation chamber 140 to the main chamber 130, a second channel 182 connecting the main chamber 130 to the sample chamber 150, a third channel 184 connecting the sample chamber 150 to the separation chamber 160, and a fourth channel 186 connecting the separation chamber 160 to the storage chamber 170.

First, the main chamber 130 of the present embodiment is provided in a central portion of the separation disk body 121, stores whole blood and a first density gradient medium (DGM) to be separated from each other, and includes layers of plasma, peripheral blood mononuclear cells (PBMCs), the DGM, and red blood cells (RBCs) which may be formed by the centrifugal force generated when the disk body 110 rotates. The main chamber 130 may include a whole blood storage space 131 in which whole blood flows and is stored, and a material storage space 135 which is partitioned by a partition wall from the whole blood storage space 131 and in which the first density gradient material flows and is stored.

With the configuration of the main chamber 130, whole blood in the whole blood storage space 131 and the first density gradient material in the material storage space 135 may be divided into blood layers composed of plasma, peripheral blood mononuclear cells, the first density gradient material, and red blood cells by centrifugal force generated when the disk body 110 rotates.

The plasma separation chamber 140 may be connected to one side of the main chamber 130 by the channel 180, and may receive and store plasma from the main chamber 130 after being centrifugally separated. The channel 180 connecting the main chamber 130 to the plasma separation chamber 140 includes an on-off valve, and accordingly, when an electromagnetic wave or so on is applied to the on-off valve by using the valve actuator 400 described above, the on-off valve may be opened, and accordingly, plasma may move from the main chamber 130 to the plasma separation chamber 140.

Also, the sample chamber 150 is connected to the main chamber 130 to face the plasma separation chamber 140 with the main chamber 130 as the center, and peripheral blood mononuclear cells that are separated from the blood layer formed after centrifugation may be stored in the sample chamber 150. Also, a channel 182 connecting the main chamber 130 to the sample chamber 150 includes an on-off valve 185, and when an electromagnetic wave or so on is applied to the on-off valve 185 by using an electromagnetic wave generator or so on, the on-off valve 185 may be opened, and accordingly, the peripheral blood mononuclear cells may move from the main chamber 130 to the sample chamber 150.

Beads, which are leukocyte-labeling antibodies, such as CD45-dynabeads, are injected into the sample chamber 150 to selectively remove leukocytes mainly present in the peripheral blood mononuclear cells, and accordingly, leukocytes labeled with beads and circulating tumor cells from which leukocytes are removed may move through a channel from the sample chamber 150 to the separation chamber 160.

The separation chamber 160 of the present embodiment may include a second density gradient material, and leukocytes labeled with beads moved from the sample chamber 150 and circulating tumor cells from which leukocytes are removed may form different layers with the second density gradient material therebetween.

That is, white blood cells labeled with beads settle below the second density gradient material, and the circulating tumor cells are formed above the second density gradient material, and accordingly, the circulating tumor cells may be separated therefrom and move into the circulating tumor cell storage chamber 170 through the channel 186.

In this way, in the present embodiment, each blood separator 120 has a multichamber structure, and accordingly, target cells, that is, circulating tumor cells of the present embodiment, may be extracted accurately and efficiently from whole blood.

In addition, in the present disclosure, a lower volume of the main chamber 130 is specifically specified, and accordingly, a target material, particularly a mononuclear cell layer, may be accurately separated.

As illustrated in FIG. 6, the main chamber 130 is formed such that a preset volume is determined by a rim 132 of the main chamber, and when the main chamber 130 is cut parallel to a horizontal plane, the rim 132 of the main chamber has a cross-section of a preset shape. In addition, the second channel 182 is connected to one side surface of the main chamber 130.

In addition, when viewed from a cross-section of the main chamber 130, a lower volume of the main chamber 130, which is determined by a height from a lower end 133 of the main chamber 130 to a point connected to the second channel 182, is formed to be less than or equal to the sum of a volume of a red blood cell layer of corresponding blood and a volume of a density gradient solution under a condition that a mixture of blood and density gradient solution corresponding to the entire volume of the main chamber 130 is injected. In other words, the present disclosure has characteristics in that a ratio of the lower volume to a total volume of the main chamber 130 is specifically specified. When a volume of the mixture of blood and density gradient solution that may fill entirety of the main chamber 130 is defined as the total volume of the main chamber 130, the lower volume of the main chamber 130 is designed to be less than or equal to the sum of the volume of the red blood cell layer of the blood and the volume of the density gradient solution in the mixture.

In the main chamber 130, layers are separated in order of density from a lower end of the main chamber 130 by a density gradient. That is, a red blood cell layer (d ≥ 1.09) with the highest density, a density gradient solution layer (1.077 ≤ d ≤ 1.084) with the next highest density, a mononuclear cell layer (1.067 ≤ d ≤ 1.077), and a plasma layer (d ≒ 1.05) are formed in this order, and a layer with the highest density is located on a lower end side of the main chamber 130.

In addition, the main chamber 130 is formed according to conditional equation 1 below. Lower volume of main chamber ≤ Sum of volume of red blood cell layer and volume of density gradient solution in mixture of blood and density gradient solution corresponding to total volume of main chamber

In this case, the volume of the red blood cell layer may be calculated by multiplying a hematocrit value by a volume of the entire blood, and represents a ratio of a volume of a red blood cell to a volume of the entire blood, and is generally less than 60%.

Therefore, conditional equation 1 may be modified as conditional equation 2 below. Lower volume of main chamber ≤ Sum of 60% of blood volume and volume of density gradient solution among mixture of blood and density gradient solution corresponding to total volume of main chamber

A material separation disk of FIG. 7 is entirely different in shape from the material separation disk of FIG. 3, and includes a plasma separation chamber 140', a main chamber 130', a sample chamber 150', a first channel 180', and a second channel 182'. Also, as described above, a lower volume of the main chamber 130' is formed to satisfy Condition Equation 1 or Condition Equation 2 described above. That is, when a volume of a mixture of blood and density gradient solution that may fill the entire main chamber 130' is defined as a total volume of the main chamber 130', a lower volume of the main chamber 130' is designed to be less than or equal to the sum of a volume of a red blood cell layer of blood and a volume of a density gradient solution in the mixture, or is designed to be less than or equal to the sum of 60% of a volume of blood and a volume of the density gradient solution in the mixture.

Also, the present disclosure clearly specifies a shape of the sample chamber 150. Monocytes transferred to the sample chamber 150 are sufficiently mixed with microbeads and then move to the separation chamber 160 via the third channel 184. In order to move objects (cells, microbeads, and so on) in the sample chamber 150 to the separation chamber 160 via the third channel 184, a sufficient angle of inclination is required to counteract centrifugal force.

Referring to FIG. 8, a part of an outer peripheral surface of the sample chamber 150 has a shape where a first outer peripheral line 151 meets a second outer peripheral line 152, and when a third outer peripheral line 153 is taken into consideration, the sample chamber 150 may have a shape in the form of an inverted triangle. In addition, the third channel 184 is connected to a vertex where the first outer peripheral line 151 is connected to the second outer peripheral line 152. In this case, the third channel 184 extends vertically downward from the vertex where the first outer peripheral line 151 is connected to the second outer peripheral line 152, and as illustrated in FIG. 8, the first outer peripheral line 151 and the second outer peripheral line 152 may have a symmetrical relationship with respect to an extension line of the third channel 184. However, the present disclosure is not limited thereto, and depending on shapes of the sample chamber 150, the first outer peripheral line 151 and the second outer peripheral line 152 may have an asymmetrical relationship. In this case, a first angle θ between a first linear line 154 connecting a vertex to the other end of the first outer peripheral line 151 and a reference line 156 perpendicular to an extension line of the third channel 184 and a second angle θ' between a second linear line 155 connecting the vertex to the other end of the second outer peripheral line 152 and the reference line 156 are set to acute angles of 30° or more. In this case, the first angle θ and the second angle θ' may be set differently.

In this case, it is preferable that an angle α between the first linear line 154 and the extension line of the third channel 184 and is set to be equal to an angle α' between the second linear line 155 and the extension line of the third channel 184. This also means that the angle θ between the first linear line 154 and the reference line 156 perpendicular to the extension line of the third channel 184 has the same condition as the angle θ' between the second linear line 155 and the reference line 156.

However, even when the respective angles are not equal to each other, a difference (α - α') between the respective angles is set to be 60° or less.

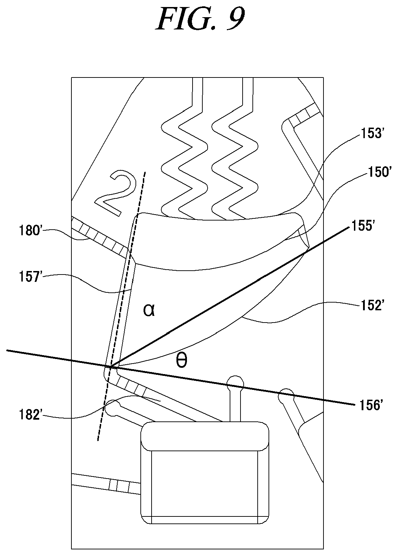

In addition, the sample chamber 150' having a deformed shape like the material separation disk of FIG. 7 described above satisfies the following conditions. FIG. 9 illustrates a configuration of a sample chamber of a material separation disk according to another embodiment of the present disclosure.

A sample chamber 150' has a shape in which the sample chamber 150 of FIG. 3 is cut by half based on an extension line of the third channel 184. That is, when the sample chamber 150 of FIG. 3 has a shape similar to an isosceles triangle, the sample chamber 150' has a shape similar to a right triangle folded in half. This sample chamber 150' has a shape in which a first linear line 157', a first outer peripheral line 152', and a second outer peripheral line 153' are connected in a shape similar to a right triangle. A second channel 180' is connected to one end of the first linear line 157', and a third channel 182' is connected to a vertex where the other end of the first linear line 157' meets the first outer peripheral line 152'. In this case, when considering a reference line 156' that is perpendicular to the first linear line 157' and passes through the vertex, an angle θ between a second linear line 155' connecting the vertex to the other end of the first outer peripheral line 152' and the reference line 156' is set to an acute angle of 30° or more. In other words, an angle α between the first linear line 157' and the second linear line 155' is set to an acute angle less than 60°.

Referring again to FIG. 1, the material separation device 10 further includes the third motor 700 that rotates the valve actuator 400. The third motor 700 rotates the valve actuator 400 around a rotation center RC2 that is coaxial with a rotation center RC1 of the material separation disk 100. That is, the rotation centers RC1 and RC2 are located on a common rotation axis AX. The third motor 700 rotates the valve actuator 400 in synchronization with the rotation of the material separation disk 100. The third motor 700 rotates the rotation member 720. In this case, the second motor 600, the guide rail 640, and the lead screw 630 described above may be installed in a lower portion of the rotation member 720. Although FIG. 1 illustrates that the third motor 700 is directly connected to the rotation member 720, the third motor 700 may also be connected to the rotation member 720 by a power connection element, such as a belt, chain, or gear.

The controller 800 controls the first, second, and third motors 500, 600, and 700 and controls the entire sample analysis process. The controller 800 may include a memory 830, a motor driver 820 that drives the first to third motors 500, 600, and 700, and a central processing unit 810. The memory 830 may store application software for controlling a sample analysis process. Also, the memory 830 may store position coordinate values of the plurality of valves A provided on the material separation disk 100, for example, polar coordinate values r and θ based on the rotation center RC1 and the first reference line L1. The application software and the position coordinate values of the plurality of valves A may be downloaded from a host computer connected to the material separation device 10 and stored in the memory 830. Also, the position coordinate values of the plurality of valves A may be directly input to the memory 830 by a user through an input device not illustrated.

The first motor 500 may be a servo motor. The servo motor includes an encoder, which may count the number of rotations, and a feedback mechanism for rotation control. Therefore, a rotation phase of the first motor 500, for example, an angular position of a reference position of the encoder with respect to a control reference polar coordinate system of the material separation device 10 may be known at any time. When the material separation disk 100 is mounted on the first motor 500, the reference position of the encoder of the first motor 500 is aligned with a first reference line L1 of the material separation disk 100. Then, the angular position of the valve A with respect to the control reference polar coordinate system of the material separation device 10 may be known at any time. For example, a first alignment portion 103 aligned with the first reference line L1 may be provided in the mounting hole 102 of the material separation disk 100, and a second alignment portion 521 coupled to the first alignment portion 103 and aligned with the reference position of the encoder of the first motor 500 may be provided in the turntable 520. The first alignment portion 103 and the second alignment portion 521 may have complementary shapes. For example, the first alignment portion 103 may have a shape of a groove extending outward from the mounting hole 102 having a circle, and the second alignment portion 521 may have a shape of a protrusion inserted into the first alignment portion 103.

The third motor 700 may be a servo motor. A second reference line L2 of the rotation member 720 may be aligned with a reference position of an encoder built in the third motor 700. The second reference line L2 may coincide with a radial movement trajectory of the valve actuator 400. Also, the second reference line L2 may also have a phase difference of θ₀ from the radial movement trajectory of the valve actuator 400.

Also, referring to FIGS. 1 and 2, the material separation device 10 may further include a first phase detector 851 and a second phase detector 852. The first phase detector 851 and the second phase detector 852 may respectively detect a first phase pattern P1 and a second phase pattern P2 provided respectively in the material separation disk 100 and the rotation member 720. The first phase pattern P1 and the second phase pattern P2 are respectively aligned with the first reference line L1 of the material separation disk 100 and the second reference line L2 of the rotation member 720. The controller 800 may calculate rotational speeds of the material separation disk 100 and the rotation member 720, angular positions of the first reference line L1 and the second reference line L2, and angular positions of the plurality of valves A and the valve actuator 400 at any time based on output signals (first and second phase signals) of the first phase detector 851 and the second phase detector 852. The first phase pattern P1 and the second phase pattern P2 may be, for example, optically recognizable patterns or magnetically recognizable patterns. The first phase pattern P1 may be aligned with the first alignment portion 103.

### Sample Processing

The controller 800 drives the first motor 500 to rotate the material separation disk 100 at a first speed (V1) to perform a sample processing process, such as centrifugation of a sample and mixing of the sample with an additive depending on analysis purposes. For example, the material separation disk 100 is loaded with a sample and rotated to perform centrifugation of the sample. Then, as needed, an operation for opening the plurality of valves A is performed to transfer some or all of the centrifuged material layers to another chamber. During the sample processing process, the rotation member 720 may be maintained in a stationary state without rotation.

### Radial Position Alignment

The controller 800 drives the second motor 600 to align a radial position of the valve actuator 400 with a radial position of the valve A. To this end, the controller 800 reads radial coordinates r of the valve A from the memory 830. The controller 800 may detect a variation of a current for driving the second motor 600 while driving the second motor 600, and recognize a position, in which the variation is detected, as a reference position r₀ in a radial direction of the valve actuator 400. According to another method, the controller 800 drives the second motor 600 to move the movement member 620 in a radial direction, and detects the movement member 620 by using the position detector 650. When the movement member 620 is detected, the controller 800 recognizes that the valve actuator 400 is located at the reference position r₀ in the radial direction. The controller 800 determines a rotation amount and a rotation direction of the second motor 600, which correspond to a value of r - r₀, and drives the second motor 600 based on the rotation amount and the rotation direction to move the valve actuator 400 in the radial direction. A process of the radial position alignment may be performed in advance while the rotation member 720 stops without rotation while the sample processing process is performed as described above.

### Angular Position Alignment

Next, the controller 800 drives the third motor 700 to rotate the valve actuator 400 in synchronization with the material separation disk 100. The controller 800 reads the angular position θ of the valve A from the memory 830. The controller 800 drives the third motor 700 through the motor driver 820. The controller 800 adjust a rotation speed of the rotation member 720 and a rotation speed of the material separation disk 100 by controlling the third motor 700 based on an encoder output signal (a first encoder output signal) of the third motor 700 or an output signal (a second phase signal) of the second phase detector 852.

Then, the controller 800 calculates angular positions of the valve actuator 400 and the valve A with respect to a reference polar coordinate system at any time from encoder output signals (first and second encoder output signals) of the first motor 500 and the third motor 700 or output signals (first and second phase signals) of the first phase detector 851 and the second phase detector 852, and determines a difference between the two angular positions. The controller 800 performs a feedback-control of a rotation speed of the third motor 700 based on the difference, and thereby, the angular positions of the valve actuator 400 and the valve A with respect to the reference polar coordinate system coincide with each other. Accordingly, the valve actuator 400 rotates in synchronization with the material separation disk 100.

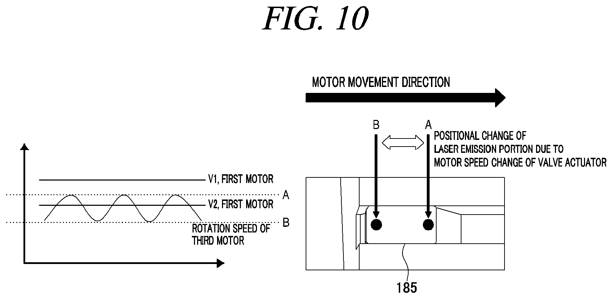

FIG. 10 illustrates a rotation speed of the material separation device 100 according to an embodiment of the present disclosure.

As illustrated in FIG. 10, when the material separation disk 100 rotates at a first rotation speed V2 lower than a rotation speed (V1), the controller 800 alternately rotates the third motor 700 at a second rotation speed and a third rotation speed. In this case, the second rotation speed is less than the first rotation speed V2, and the third rotation speed has a value greater than or equal to the first rotation speed V2.

In addition, starting timing of the third motor 700 may be determined such that the valve actuator 400 is located above the valve A on the material separation disk 100 at a point in time when a rotation speed of the rotation member 720 is equal to the first rotation speed, that is, such that angular positions of the valve actuator 400 and the valve A are aligned with each other. In this way, the rotation speed of the rotation member 720 rotated by the third motor 700 is changed continuously and alternately between the second rotation speed and the third rotation speed, and when the rotation speed of the third motor 700 is equal to the first rotation speed of the first motor 500, the angular positions are considered to be aligned, and accordingly, the valve actuator 400 operates.

In addition, as illustrated in FIG. 10, a valve material included in the on-off valve 185 has a preset area, and when the entire area where the valve material is spread is called an entire valve area, and when the material separation disk 100 rotates at the first rotation speed V2, the controller 800 alternately rotates the third motor 700 between the second rotation speed and the third rotation speed, and accordingly, the valve actuator 400 may move to positions A and B among positions of the entire valve area to supply energy (for example, laser). That is, as illustrated, assuming that the material separation disk 100 rotates in a right direction, and when the third motor 700 rotates at the second rotation speed, the valve actuator 400 emits the laser to a first point B located slightly behind the center of the valve material based on a rotation direction of the material separation disk 100, and when the third motor 700 rotates at the third rotation speed, the valve actuator 400 emits the laser to a second point A located slightly ahead of the center of the valve material. In other words, respective rotation speeds are adjusted such that a first virtual line connected to the first point B on a rotation axis is located, by a preset rotation angle, behind a reference line from the rotation axis of the material separation disk 100 toward the center of the valve material, and such that a second virtual line connected to the second point A on the rotation axis is located ahead of the reference line by a preset rotation angle, based on a rotation direction of the material separation disk 100.

In this way, the valve actuator 400 may move with respect to the valve material and efficiently emit energy.

### Valve Operation

When the valve actuator 400 is located at an upper portion of a certain valve by the radial position alignment and the angular position alignment described above, the controller 800 drives the valve actuator 400 to emit, for example, a laser beam to the valve material. Then, the energy of the laser beam is absorbed by the valve material, and accordingly, the valve material is melted. Because the material separation disk 100 is in a rotating state, the melted valve material is pushed out of a channel C by centrifugal force, and accordingly, the valve A may be opened.

As described above, by operating the valve A while the valve actuator 400 rotates together with the material separation disk 100, the channel C may be opened or closed by centrifugal force before the molten valve material is solidified, and thus, the operational reliability and speed of the valve A may be improved. Also, because the material separation disk 100 rotates continuously while the valve A operates, a centrifuged sample may be maintained in a separated state, and thus, separated layers may be prevented from being mixed together. Also, because a process of operating the valve A may be performed without a process of stopping the rotation of the material separation disk 100, the time required for analyzing samples may be reduced.

The description of the present disclosure made above is for illustrative purposes only, and those skilled in the art will appreciate that the present disclosure may be easily modified into other specific forms without changing the technical idea or essential characteristics of the present disclosure. Therefore, the embodiments described above should be understood as illustrative in all respects and not limiting. For example, the components described in a single type may also be implemented in a distributed manner, and likewise, the components described in the distributed manner may be implemented in a combined manner.

The scope of the present application is indicated by the claims described below rather than the detailed description above, and all changes or modified forms derived from the meaning, scope of the claims, and their equivalent concepts should be interpreted as being included in the scope of the present application.

### [Description of Symbols]

- 10 :: material separation device
- 100 :: material separation disk
- 110 :: disk body
- 120 :: blood separator
- 121 :: modular separation disk body
- 130 :: main chamber
- 131 :: whole blood storage space
- 135 :: material storage space
- 140 :: plasma separation chamber
- 150 :: sample chamber
- 160 :: separation chamber
- 170 :: storage chamber
- 180 :: channel
- 185 :: valve for opening and closing
- 400 :: valve actuator
- 500 :: first motor
- 600 :: second motor
- 700 :: third motor
- 800 :: controller

## Claims

1. A material separation device comprising:
a first motor configured to rotate a material separation disk including a chamber that is a sample storage space, a channel providing a passage for flow of a sample, and a valve for selectively opening and closing the channel;
a valve actuator configured to supply energy for operating the valve;
a third motor configured to rotate the valve actuator based on a coaxial axis and the first motor; and
a controller configured to control the first motor, the third motor, and the valve actuator to supply energy to a valve material included in the valve while the material separation disk and the valve actuator rotate at different rotational speeds.

2. The material separation device of claim 1, wherein
the controller is further configured to control the first motor and the third motor to supply energy to an entire region of the valve material by rotating the material separation disk at a first rotation speed through the first motor while the valve is operated by the valve actuator and by alternately rotating the valve actuator at a second rotation speed and a third rotation speed,
the second rotation speed is less than the first rotation speed, and
the third rotation speed is greater than or equal to the first rotation speed.

3. The material separation device of claim 2, wherein
the controller is further configured to rotate the valve actuator at the first rotation speed to supply energy to a center of the valve material, rotate the valve actuator at the second rotation speed to supply energy to a first point in the entire region of the valve material, and rotate the valve actuator at the third rotation speed to supply energy to a second point in the entire region of the valve material, and
a first virtual line connected to the first point on a rotation axis is located, by a preset rotation angle, behind a reference line from a rotation axis of the material separation disk toward a center of the valve material, and a second virtual line connected to the second point on the rotation axis is located ahead of the reference line by a preset rotation angle, based on a rotation direction of the material separation disk.

4. The material separation device of claim 1, wherein
the material separation disk comprises a disk body that is rotatable; and at least one blood separator arranged on the disk body, including a plasma separation chamber, a main chamber, and a sample chamber, and including a first channel connecting the plasma separation chamber to the main chamber and a second channel connecting the main chamber to the sample chamber, and
the main chamber has a cross-section of a preset shape when being cut parallel to a horizontal plane and has one side surface connected to the second channel, and
a lower volume of the main chamber, which is determined by a height from a lower end of the cross-section of the main chamber to a point connected to the second channel, is formed to be less than or equal to a sum of a volume of a red blood cell layer of corresponding blood and volume of a density gradient solution under a condition that a mixture of the density gradient solution and blood corresponding to an entire volume of the main chamber is injected.

5. The material separation device of claim 4, wherein
the disk body includes at least one coupling groove, and
the at least one blood separator is formed to have a shape of a modular separation disk to be coupled to the at least one coupling groove of the disk body.

6. The material separation device of claim 4, wherein
the lower volume of the main chamber is formed to be less than or equal to a sum of 60% of the volume of the blood and the volume of the density gradient solution under the condition that the mixture of the density gradient solution and the blood corresponding to the entire volume of the main chamber is injected.

7. The material separation device of claim 4, wherein
the at least one blood separator further includes a separation chamber and further includes a third channel connecting the sample chamber to the separation chamber.

8. The material separation device of claim 7, wherein
at least part of an outer periphery of the sample chamber has a shape in which a first outer peripheral line meets a second outer peripheral line,
the third channel is connected to a vertex in which one end of the first outer peripheral line is connected to one end of the second outer peripheral line, and
a first angle between a first linear line connecting the vertex to another end of the first outer peripheral line and a reference line perpendicular to an extension line of the third channel and a second angle between a second linear line connecting the vertex to another end of the second outer peripheral line and the reference line are each set to an acute angle of 30° or more.

9. The material separation device of claim 7, wherein
the first outer peripheral line and the second outer peripheral line are symmetric to an extension line of the third channel.

10. The material separation device of claim 7, wherein
at least part of an outer periphery of the sample chamber has a shape in which a first linear line meets a second linear line,
the third channel is connected to a vertex in which one end of the first linear line is connected to one end of the second linear line,
the first linear line and the second linear line are symmetric to an extension line of the third channel, and
a first angle between the first linear line and the reference line perpendicular to the extension line of the third channel and a second angle between the second linear line and the reference line are each set to an acute angle of 30° or more.

11. The material separation device of claim 8, wherein
a difference between an angle between the first linear line and the extension line of the third channel and an angle between the second linear line and the extension line of the third channel is 60° or less.

12. The material separation device of claim 7, wherein
the sample chamber has a shape in which a first linear line, a first outer peripheral line, and a second outer peripheral line are connected to each other in a form of a right triangle,
the second channel is connected to one end of the first linear line,
the third channel is connected to a vertex in which another end of the first linear line meets the first outer peripheral line, and
an angle between a second linear line connecting the vertex to another end of the first outer peripheral line and a reference line that is perpendicular to the first linear line and passes through the vertex is set to an acute angle of 30° or more.

13. The material separation device of claim 7, wherein
the at least one blood separator further includes a storage chamber and further includes a fourth channel connecting the separation chamber to the storage chamber.

14. The material separation device of claim 4, wherein
the disk body has at least one fixing groove into which a fixing pin formed on a turntable of the material separation device is inserted and to which the fixing pin is coupled, or includes at least one fixing pin formed to be inserted into and coupled to the at least one fixing groove formed on the turntable of the material separation device.
